# EUROPEAN PATENT APPLICATION

(11) **EP 3 811 980 A1**
(43) Date of publication of application: **28.04.2021**
(21) Application number: 19822139.2
(22) Date of filing: 19.06.2019
(51) Int. Cl.: A61K 51/08, A61K 38/08, A61K 38/10, A61K 47/64, A61P 35/00, C07K 7/06, C07K 7/08

(54) **ACCUMULATIVE BORON 10 MEDICINE FOR BORON NEUTRON CAPTURE THERAPY FOR SELECTIVELY OR LOCALLY TARGETING TUMOR TISSUES IN SHORT TIME**

(30) Priority: 20.06.2018 JP 2018117189
(71) Applicant: HIROSAKI UNIVERSITY, Hirosaki-shi, Aomori 036-8560 (JP)
(72) Inventor: OHYAMA, Chikara, Hirosaki-shi, Aomori 036-8560 (JP); YONEYAMA, Tohru, Hirosaki-shi, Aomori 036-8560 (JP); HATAKEYAMA, Shingo, Hirosaki-shi, Aomori 036-8560 (JP); ISHIYAMA Shintaro, Hirosaki-shi, Aomori 036-8560 (JP)
(74) Representative: Appleyard Lees IP LLP
(86) International application number: PCT/JP2019/024367
(87) International publication number: WO 2019/244954

(57) **Abstract**

The purpose of the present invention is to provide a ¹⁰B medicine that can be selectively accumulated in tumor tissues at a low dose in a short time, and can be applied to BNCT. The ¹⁰B medicine comprises a compound containing: a peptide capable of selectively binding to tumor vascular endothelial cells; and ¹⁰B, wherein the ¹⁰B medicine is administered to a subject suffering from cancer at a dose of 300-600mg per administration, and is accumulated after the administration such that the concentration of ¹⁰B in the cancer tissue of the subject becomes 1ppm or higher.

SEQUENCE LISTING

<110>  Hirosaki University

<120>  Anti-cancer agent

<130>  PCT19-001

<150>  JP 2018-117189
<151>  2018-06-20

<160>  5

<170>  PatentIn version 3.5

<210>  1
<211>  12
<212>  PRT
<213>  Artificial Sequence

<220>
<223>   chemically synthesized; annexin-1 binding compound


<220>
<221>  misc_feature
<222>  (8)..(12)
<223>  Xaa can be any naturally occurring amino acid

<400>  1

Ile Phe Leu Leu Trp Gln Arg Xaa Xaa Xaa Xaa Xaa
1               5                   10


<210>  2
<211>  10
<212>  PRT
<213>  Artificial Sequence

<220>
<223>  chemically synthesized; annexin-1 binding compound

<400>  2

Ile Phe Leu Leu Trp Gln Arg Cys Arg Arg
1               5                   10


<210>  3
<211>  10
<212>  PRT
<213>  Artificial Sequence

<220>
<223>  chemically synthesized; annexin-1 binding compound

<400>  3

Ile Phe Leu Leu Trp Gln Arg Lys Arg Arg
1               5                   10

<210>  4
<211>  9
<212>  PRT
<213>  Artificial Sequence

<220>
<223>  chemically synthesized; annexin-1 binding compound

<400>  4

Ile Phe Leu Leu Trp Gln Arg Cys Arg
1               5


<210>  5
<211>  12
<212>  PRT
<213>  Artificial Sequence

<220>
<223>  chemically synthesized; annexin-1 binding compound

<400>  5

Ile Phe Leu Leu Trp Gln Arg Cys Arg Arg Arg Arg
1               5                   10

## Description

### TECHNICAL FIELD

The present invention relates to an accumulative ¹⁰B drug which can selectively or locally target a tumor tissue in a short period of time, and which may be used in boron neutron capture therapy.

### BACKGROUND ART

The problem of current anticancer drug treatment resides in systemic administration of highly cytotoxic drugs. In order to reduce side effects such as cytotoxicity and obtain a high anticancer effect, it is important to deliver a high concentration of an anticancer drug to only the cancer in as short a time as possible. On the other hand, boron neutron capture therapy (BNCT) uses the fact that neutron irradiation of a boron-10 nuclei (boron 10; ¹⁰B), which has a large reactive cross-sectional area with neutrons, generates secondary nuclei each having a short range (in the order of micrometers) and the secondary nuclei selectively destroy cancer cells in the vicinity. Since the BNCT is expected to have a high and local auto-destruction effect to cancer cells in the range of several micrometers, targeting of cancer tissue is a problem to be solved. Thus, whether BNCT can be effectively performed is dependent on how selectively ¹⁰B is taken up by cancer cells: for example, in a tumor nest arising at an unresectable location in an organ, BNCT is expected to selectively kill cancer cells with minimal damage to the organ, and to be applied to tumors that are unresectable with surgical treatment. Since neutrons reach a depth of only 7 to 8 cm in the human body, it is expected that a higher uptake efficiency of ¹⁰B into cancer cells will result in a broader range of cancers and organs becoming treatable as well as an improvement in cancer therapy effect by BNCT.

On the other hand, Non-Patent Document 1 discloses that the authors thereof searched for a peptide that specifically binds to tumor vasculature and does not target normal lung vasculature, using a phage library, obtained a peptide with an amino acid sequence IFLLWQR (which may be hereinafter simply referred to as "IF7"), and confirmed that IF7 phage binds to recombinant annexin 1; and that IF7 which was bound with a geldanamycin derivative suppressed tumor growth.

In addition, Patent Document 1 discloses a composition containing a component for cancer therapy which is covalently bonded directly or via a linker to a carboxy terminal end including IF7. Specifically, Patent Document 1 discloses anticancer activity by a composition containing a compound obtained by binding geldanamycin to IF7 or a composition containing a compound obtained by binding an anticancer drug SN-38 to IF7.

Patent Document 1: Japanese Patent No. 6184101

Non-Patent Document 1: Proc Natl Acad Sci USA., Vol. 108, No. 49, Pages 19587-19592 (2011)

### DISCLOSURE OF THE INVENTION

### Problems to be Solved by the Invention

However, the compound obtained by binding geldanamycin to IF7 or the compound obtained by binding SN-38 to IF7 which are disclosed in Patent Document 1 can be applied only to a particular cancer (e.g., colon cancer), and a satisfactory anticancer activity could not be obtained when the compounds were applied to a wide variety of types of cancer. Moreover, anticancer drugs other than geldanamycin and SN-38 were bound to IF7 but anticancer activity was not obtained. This was considered to be for the following reasons: that anticancer drugs alter the conformation thereof when bound to IF7; and that although the anticancer drugs each were of such a type whose anticancer activity is exerted after the anticancer drug is metabolized and its structure is changed, the anticancer drug was bound to IF7; this prevented the anticancer drug from being metabolized, resulting in no change in the structure; and as a result anticancer activity could not be obtained. Therefore, the invention described in Patent Document 1 cannot be easily applied to a wide variety of cancer types and the anticancer activity thereof is also restricted. In addition to such problems, development of ¹⁰B drugs with respect to conventional drugs requires (1) local accumulation of ¹⁰B and (2) accumulation of ¹⁰B in a short period of time at the same time, in order to make the invention of Patent Document 1 exert an epoch-making therapeutic effect against refractory cancers in BNCT therapy.

The present invention has been made in view of the above-mentioned problems of prior art, and it is an object of the present invention to provide a ¹⁰B drug which can selectively accumulate into tumor tissue in a short period of time at a low dosage amount and which can be applied to BNCT.

### Means for Solving the Problems

The inventors have found that a compound containing ¹⁰B and a peptide which can selectively bind to a tumor vascular endothelial cell can rapidly and selectively accumulate in tumor tissue at a low dosage amount. The inventors applied the compound to BNCT and have found that the compound has less side effects, is minimally invasive and achieves anticancer effects selectively to tumor tissue. In addition, the inventors have also found that when applied to BNCT, the above-mentioned compound reaches the tumor via tumor vascular endothelial cells; therefore, not only ¹⁰B which is taken up by the tumor itself, but also ¹⁰B which is taken up by the tumor blood vessels or cells can destroy the tumor blood vessels; and thereby tumor growth is indirectly inhibited. The present invention has been completed based on these findings. That is, the present invention is as follows.

A first aspect of the present invention relates to an accumulative ¹⁰B drug comprising a compound comprising ¹⁰B and a peptide capable of selectively binding to a tumor vascular endothelial cell, in which the accumulative ¹⁰B drug is administered to a subject affected with cancer in an amount from 300 and 600 mg/administration and in which the ¹⁰B drug accumulates in a cancer-affected tissue of the subject after the administration so that a ¹⁰B level therein is 1 ppm or more.

A second aspect of the present invention relates to the accumulative ¹⁰B drug as described in the first aspect, in which the accumulative ¹⁰B drug accumulates so that the ¹⁰B level in the cancer-affected tissue of the subject is 20 ppm or more.

A third aspect of the present invention relates to the accumulative ¹⁰B drug as described in the first or second aspect, in which the ¹⁰B level in the cancer-affected tissue is 20 ppm or more during a period from 10 minutes to 30 minutes after the administration of the accumulative ¹⁰B drug.

A fourth aspect of the present invention relates to the accumulative ¹⁰B drug as described in any one of the first to third aspects, in which the administration is injection administration.

A fifth aspect of the present invention relates to the accumulative ¹⁰B drug as described in any one of the first to fourth aspects, in which the compound contains a ¹⁰B-containing group and the ¹⁰B-containing group is an L-p-[10B]boronophenylalanine group, an fluoro[10B]boronophenylalanine group, or a borocaptate group.

A sixth aspect of the present invention relates to the accumulative ¹⁰B drug as described in any one of the first to fifth aspects, in which the peptide is a peptide capable of selectively binding to annexin 1.

A seventh aspect of the present invention relates to an accumulative ¹⁰B drug, comprising a ¹⁰B-containing group and a peptide that can selectively bind to annexin 1, in which the ¹⁰B-containing group is an L-p-[10B]boronophenylalanine group, an [18F]fluoro[10B]boronophenylalanine group, or a [10B]borocaptate group.

An eighth aspect of the present invention relates to the accumulative ¹⁰B drug as described in the sixth or seventh aspect, in which the peptide comprises an amino acid sequence of IFLLWQR (amino acids 1 to 7 of SEQ ID NO: 1), an amino acid sequence of IFLLWQRX (amino acids 1 to 8 of SEQ ID NO: 1), an amino acid sequence of IFLLWQRXX (amino acids 1 to 9 of SEQ ID NO: 1), an amino acid sequence of IFLLWQRXXX (amino acids 1 to 10 of SEQ ID NO: 1), an amino acid sequence of IFLLWQRXXXX (amino acids 1 to 11 of SEQ ID NO: 1), or an amino acid sequence of IFLLWQRXXXXX (amino acids 1 to 12 of SEQ ID NO: 1), in which each X independently represents a polar or charged amino acid, and in which one or two amino acids of IFLLWQR in each of the amino acid sequences may be substituted.

A ninth aspect of the present invention relates to the accumulative ¹⁰B drug as described in any one of the first to eighth aspects, in which the peptide and a ¹⁰B-containing group are linked to each other via a linker.

A tenth aspect of the present invention relates to the accumulative ¹⁰B drug as described in the ninth aspect, in which the ¹⁰B-containing group is an L-p-[10B]boronophenylalanine group or an [18F]fluoro[10B]boronophenylalanine group, and in which the ¹⁰B-containing group is linked to the linker via an ester bond or the linker contains an ester bond.

An eleventh aspect of the present invention relates to the accumulative ¹⁰B drug as described in the ninth aspect, in which the ¹⁰B-containing group is a [10B]borocaptate group and in which the linker contains no ester bond.

A twelfth aspect of the present invention relates to the accumulative ¹⁰B drug as described in any one of the ninth to eleventh aspects, in which the linker is a linker represented by the following formula (i) or (ii): 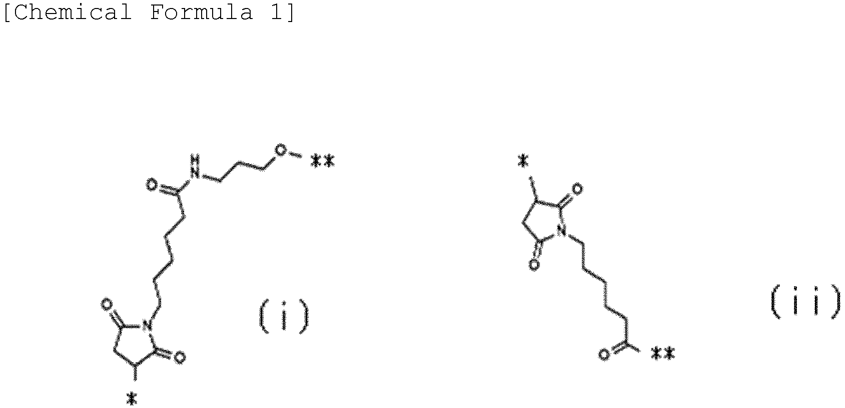 in which * and ** each represent a bond.

A thirteenth aspect of the present invention relates to the accumulative ¹⁰B drug as described in any one of the first to twelfth aspects, in which the compound is a compound containing a structure represented by the following formula (i-1) or (ii-1) : 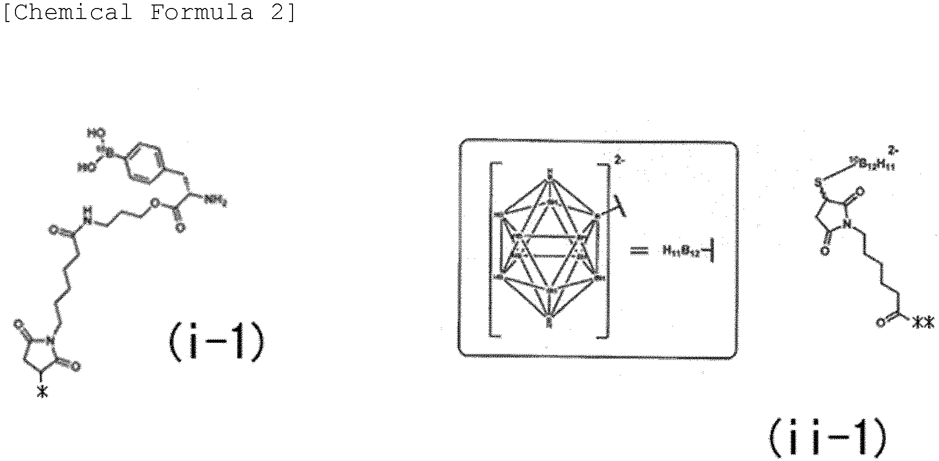 in which * and ** each represent a bond at which the peptide capable of selectively binding to a tumor vascular endothelial cell binds.

A fourteenth aspect of the present invention relates to the accumulative ¹⁰B drug as described in any one of the first to thirteenth aspects, in which the ¹⁰B drug is for use in BNCT.

A fifteenth aspect of the present invention relates to the accumulative ¹⁰B drug as described in the fourteenth aspect, in which the ¹⁰B drug is used to perform neutron irradiation within 60 minutes after the administration.

A sixteenth aspect of the present invention relates to the accumulative ¹⁰B drug as described in the fourteenth or fifteenth aspect, in which the ¹⁰B drug is used to perform neutron irradiation at a dose of 2 × 10⁶/cm² s or more.

The present invention also relates to the following.

A seventeenth aspect of the present invention relates to the accumulative ¹⁰B drug as described in the first aspect, in which a dosage amount per administration to the subject is 5 to 9 mg per unit body weight (1kg) of the subject.

An eighteenth aspect of the present invention relates to a cancer therapeutic drug comprising the accumulative ¹⁰B drug as described in any one of the first to seventeenth aspects.

A nineteenth aspect of the present invention relates to boron neutron capture therapy, comprising administering the accumulative ¹⁰B drug as described in any one of the first to seventeenth aspects at a dosage of 300 to 600 mg/administration to a cancer affected subject.

A twentieth aspect of the present invention relates to a method for treating cancer, including boron neutron capture therapy as described in the nineteenth aspect.

### Effects of the Invention

The accumulative ¹⁰B drug of the present invention can rapidly and selectively accumulate in tumor tissue at a low dosage amount (preferably in a high concentration) and is, therefore, suitable for BNCT. The accumulative ¹⁰B drug of the present invention can reduce a dose of neutrons to be irradiated to normal tissue which does not require neutron irradiation, has less side effects, is minimally invasive, and can locally exert a local effect of killing the tumor tissue.

### BRIEF DESCRIPTION OF THE DRAWINGS

The present invention is illustrated by the following drawings:
FIG. 1 includes drawings illustrating ¹⁰B levels (ppm) in organs of tumor-bearing mice measured 5, 10, and 20 minutes after 100 µL (175 µg) of IF7-¹⁰BPA or IF7-¹⁰BSH prepared to be 7 mg/kg was administered through the tail vein;
FIG. 2 is a drawing illustrating ¹⁰B levels (ppm) in organs of tumor-bearing mice, measured 20, 40, and 60 minutes after 100 µL (2,000 µg) of a ¹⁰BPA-fructose prepared to be 100 mg/kg was administered through the tail vein;
FIG. 3 includes drawings showing violin plots of ¹⁰B levels (ppm) in organs of cancer-bearing mice 10, 20, and 40 minutes after tail vein administration of IF7-¹⁰BPA prepared to be 7 mg/kg or ¹⁰BPA prepared to be 7 or 100 mg/kg (in the case of 100 mg/kg, measurement was performed 60 minutes after the administration);
FIG. 4 includes drawings showing violin plots of ¹⁰B levels (ppm) in organs of cancer-bearing mice measured 5, 10, 20, and 40 minutes after IF7-¹⁰BSH or ¹⁰BSH prepared to be 7 mg/kg was administered through the tail vein;
FIG. 5 includes drawings illustrating results of a hot group irradiated with neutrons 40 minutes after administration of IF7-¹⁰BSH or IF7-¹⁰BPA to cancer-bearing mice and results of a cold group which was not irradiated; and
FIG. 6 includes drawings illustrating results of a hot group irradiated with neutrons 40 minutes after administration of IF7-¹⁰BPA or ¹⁰BPA to cancer-bearing mice and results of a cold group which was not irradiated.

### PREFERRED MODE FOR CARRYING OUT THE INVENTION

Below, embodiments of the present invention are described in detail, but the present invention is not limited to the following embodiments and can be implemented within the scope of the object of the present invention with appropriate modifications. In addition, in this specification, "A to B" means that both limits, A and B, are included, unless otherwise specified.

### <<Anticancer Drugs>>

The accumulative ¹⁰B drug of the present invention includes a compound containing ¹⁰B and a peptide capable of selectively binding to a tumor vascular endothelial cell. The compound is administered to a cancer-affected subject at an amount of 300 to 600 mg/administration and accumulates after the administration so that a ¹⁰B level in a cancer-affected tissue of the subject is at least 1 ppm (preferably at least 20 ppm). The present accumulative ¹⁰B drug is suitable for BNCT, because the drug contains the compound containing ¹⁰B and a peptide capable of selectively binding to a tumor vascular endothelial cell, so that even when the dosage amount per administration is from 300 and 600 mg, which is lower than the dosage amounts of conventional ¹⁰B preparations for BNCT, the accumulative ¹⁰B drug of the present invention can accumulate so that the ¹⁰B level in the cancer-affected tissue is 1 ppm or more (preferably 20 ppm or more). Additionally, the accumulative ¹⁰B drug of the present invention reaches the tumor via tumor vascular endothelial cells when applied to BNCT; therefore, not only the ¹⁰B drug taken up by the tumor itself, but also the ¹⁰B drug taken up by the tumor blood vessels or cells can destroy the tumor blood vessels; and thereby tumor growth is indirectly inhibited. Accordingly, the accumulative ¹⁰B drug of the present invention may require accumulation of ¹⁰B in the tumor tissue at a high level (e.g., 20 ppm or more) or may not require such a high level (that is e.g., 1 to 20 ppm is satisfactory).

The subject is not particularly limited as long as the subject is affected with cancer, and examples include mammals (e.g., humans, swine, cattle, mice, rats, etc.) that are affected with cancer and humans (subjects or patients) who are affected with cancer are preferred.

A single dosage amount to the subject is preferably 300 to 600 mg, and more preferably 400 to 500 mg from the viewpoint of achieving a killing effect of cancer tissue by BNCT at a low dosage amount in order to suppress side effects. Further, when the accumulative ¹⁰B drug is administered at a low dosage amount per unit body weight (1 kg) of 5 to 9 mg, preferably 6 to 8 mg, to the subject in order to suppress side effects, the drug can accumulate so that the ¹⁰B level in the cancer-affected tissue is 1 ppm or more (preferably 20 ppm or more).

As values of ¹⁰B levels in cancer-affected tissue, values obtained by measuring under the conditions used in the Examples below by prompt-gamma-ray analysis (PGA method) are employed, unless otherwise specified. As the ¹⁰B level in the cancer-affected tissue, from the viewpoint of the killing effect of the cancer tissue by BNCT, 2 ppm or more, 3 ppm or more, 4 ppm or more, or 5 ppm or more are preferable, 6 ppm or more, 7 ppm or more, 8 ppm or more, 9 ppm or more, or 10 ppm or more is more preferable, 11 ppm or more, 12 ppm or more, 13 ppm or more, 14 ppm or more, or 15 ppm or more is still more preferable, 20 ppm or more is even more preferable, 25 ppm or more is particularly preferable, 30 ppm or more is more particularly preferable, 35 ppm or more is especially particularly preferable, and 40 ppm or more is the most preferable. The upper limit of the ¹⁰B level in cancer-affected tissue is not particularly limited as long as the effect of the present invention is not impaired, but examples of the level less than 20 ppm include 19 ppm or less, typically 18 ppm or less, and preferably 17 ppm or less. When the ¹⁰B level is 20 ppm or more, the ¹⁰B level is, for example, 200 ppm or less, typically 150 ppm or less, preferably 100 ppm or less, more preferably 80 ppm or less, most preferably 70 ppm or less, and particularly preferably 60 ppm or less.

Here, "accumulative" means that the ¹⁰B drug selectively heads for or locally exists in cancer-affected tissue rather than another tissue or normal tissue in the subject. For example, it can be mentioned that the ¹⁰B drug selectively and locally exists in cancer-affected tissue rather than in another tissue or normal tissue. The ¹⁰B drug locally exists in cancer-affected tissue at a ¹⁰B level of preferably 1.2 times or more, more preferably 1.5 times or more, still more preferably 2 times or more, even more preferably 3 times or more, and most preferably 4 times or more, relative to the ¹⁰B level in the other tissue or normal tissue. Although the upper limit is not particularly limited, examples thereof include 50 times or less, and typically 30 times or less.

From the viewpoint that the above-mentioned compound rapidly accumulates in cancer-affected tissue and BNCT can be rapidly applied, the administration preferably results in the ¹⁰B level in the cancer-affected tissue being 1 ppm or more (preferably 20 ppm or more), during a period from 5 minutes to 60 minutes (preferably 10 minutes to 50 minutes, more preferably 10 minutes to 40 minutes, more preferably 15 minutes to 30 minutes, particularly preferably 15 minutes to 25 minutes, and most preferably 15 minutes to 20 minutes) after the above-mentioned administration.

Administration method may be any of parenteral administration, inhalation administration, oral administration, and direct administration (DDD = Direct Drug Delivery). However, from the viewpoint that the compound can rapidly accumulate in cancer-affected tissue and can be quickly applied in BNCT, parenteral administration is preferable. Injection administration such as subcutaneous injection, intraperitoneal injection, intramuscular injection, etc., intravenous drip infusion, or direct administration to the affected part by endoscope or catheter is more preferable. Intravenous injection administration is the most preferable.

For the parenteral administration, forms of killing cancer tissue by the ¹⁰B drug of the present invention include aqueous or non-aqueous sterile solutions, suspensions, emulsions, and the like. Examples of non-aqueous solvents which may be included include propylene glycol, polyethylene glycol, vegetable oils (e.g., olive oil), injectable organic esters (e.g., ethyl oleate), and the like. Aqueous carriers which may be included include water, alcoholic/aqueous solutions, emulsions or suspensions (including saline, and buffering media). Parenteral vehicles include sodium chloride solutions, Ringer's dextrose, dextrose and sodium chloride, lactated Ringer, or fixed oils. Intravenous vehicles include fluids and nutritional supplements, electrolyte supplements (e.g., those based on Ringer's dextrose), and the like. Preservatives and other additives may also be present, such as, for example, antimicrobial substances, antioxidants, chelating agents, and inert gases, and the like. For oral administration, the form of the ¹⁰B drug of the present invention include powder or granules, suspensions or solutions in water or non-aqueous media, capsules, sachets, or tablets, and the ¹⁰B drug of the present invention may include thickeners, flavors, diluents, emulsifiers, dispersing aids or binders.

### <Compound containing ¹⁰B and Peptide capable of selectively binding to Tumor Vascular Endothelial Cell>

In the compound, it is preferable that an amino acid residue (preferably a side chain of the amino acid residue) at any position in the peptide capable of selectively binding to a tumor vascular endothelial cell binds to ¹⁰B directly or via a linker. As the bond, any bond may be used, such as a covalent bond, an ionic bond, a coordination bond, or a bond due to an intermolecular force, but a covalent bond is preferred because of stability of the bond.

### (¹⁰B)

The compound preferably contains a ¹⁰B-containing group. It is more preferable that an amino acid residue at any position of the peptide capable of selectively binding to a tumor vascular endothelial cell (preferably a side chain of the amino acid residue) binds directly or via a linker to the ¹⁰B-containing group. The ¹⁰B-containing group may be ¹⁰B itself, and examples thereof include a [10B]boronophenylalanine group represented by formula (a) below, a [10B]borocaptate group represented by formula (b) below, etc. From the viewpoint of imaging distribution of the anticancer drug of the present invention in the body by positron emission tomography (PET), the ¹⁰B-containing group may be an [18F]fluoro[10B]boronophenylalanine group (represented by, for example, formula (c) below), which is radiolabeled with ¹⁸F. 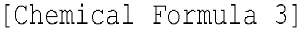 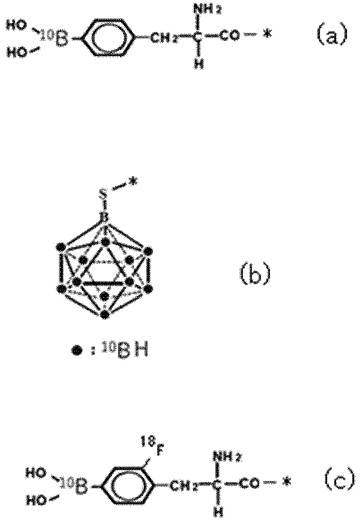 in which * represents a bond.

With respect to the embodiment in which a ¹⁰B-containing group and an amino acid residue at any position in the peptide capable of selectively binding to a tumor vascular endothelial cell are directly bound to each other, the following (1) to (5) can be exemplified but the embodiment of the present invention is not limited thereto. The [10B]boronophenylalanine group represented by the formula (a) or the [18F]fluoro[10B]boronophenylalanine group represented by the formula (c) binds to the peptide through:
(1) an amide bond with a lysine residue which may exist at any position in the peptide and which has an amino group in the side chain;
(2) a thioester bond with a cysteine residue which may exist at any position in the peptide and which has a thiol group in the side chain;
(3) an ester bond with a serine residue or a threonine residue which may exist at any position in the peptide and which has a hydroxy group in the side chain;
(4) a disulfide bond between the [10B]borocaptate group represented by the formula (b) and a cysteine residue which may exist at any position and which has a thiol group in the side chain;
(5) a thioester bond between the [10B]borocaptate group represented by the formula (b) and an aspartic acid residue or a glutamic acid residue which may exist at any position in the peptide and which has a carboxy group in the side chain; and the like. The amide bond, the thioester bond, the ester bond, and the disulfide bond can be formed by any organic chemical method.

### (Peptide capable of selectively binding to Tumor Vascular Endothelial Cell)

The peptide capable of selectively binding to a tumor vascular endothelial cell is not particularly limited, as long as it can selectively bind to a tumor vascular endothelial cell. However, a peptide capable of selectively binding to annexin 1 is preferable. Annexin 1 has been identified as a specific tumor endothelial cell surface marker and is known to be specifically expressed in tumor vascular endothelial cells (Oh, P. et al. Nature 2004; 429: pp. 629-635). The present invention relates to an anticancer drug comprising a compound comprising a ¹⁰B-containing group and a peptide capable of selectively binding to annexin 1, in which the ¹⁰B-containing group is an L-p-[10B]boronophenylalanine group or a [10B]borocaptate group.

Examples of the peptide capable of selectively binding to annexin 1 include a peptide which comprises an amino acid sequence of IFLLWQR, which has 7 to 15 amino acids (preferably 7 to 13 amino acid residues, more preferably 7 to 12 amino acid residues, and most more preferably 8 to 11 amino acid residues), and in which one or two amino acids of IFLLWQR may be substituted by any amino acid. More specifically, examples of the peptide capable of selectively binding to annexin 1 include an amino acid sequence of IFLLWQR (amino acids 1 to 7 of SEQ ID NO: 1), an amino acid sequence of IFLLWQRX (amino acids 1 to 8 of SEQ ID NO: 1), an amino acid sequence of IFLLWQRXX (amino acids 1 to 9 of SEQ ID NO: 1), an amino acid sequence of IFLLWQRXXX (amino acids 1 to 10 of SEQ ID NO: 1), an amino acid sequence of IFLLWQRXXXX (amino acids 1 to 11 of SEQ ID NO: 1), or an amino acid sequence of IFLLWQRXXXXX (amino acids 1 to 12 of SEQ ID NO: 1), in which each X independently represents a polar or charged amino acid, and in which one or two amino acids of IFLLWQR in each of the amino acid sequences may be substituted.

For example, each X may be independently selected from any set consisting of five amino acids of the amino acids C, R, K, S, T, H, D, E, N, Q, and M, any set consisting of four amino acids of all the amino acids, any set consisting of three amino acids of all the amino acids, any set consisting of two amino acids of all the amino acids, or any one of all the amino acids. For example, each X may be independently selected from a set consisting of three amino acids C, R, and K. Examples of the peptide capable of selectively binding to annexin 1 include IFLLWQRCRR (SEQ ID NO: 2), IFLLWQRKRR (SEQ ID NO: 3), IFLLWQRCR (SEQ ID NO: 4), IFLLWQRCRRRR (SEQ ID NO: 5), and the like.

The peptide may be bound directly or via a linker to ¹⁰B (preferably a ¹⁰B-containing group) at any amino acid residue of the peptide. As the binding position, a position between the sixth and twelfth amino acid residues (preferably, a side chain of the amino acid residue) in the respective amino acid sequences can be exemplified. A position between the seventh and eleventh amino acid residues is preferred, a position between the eighth and tenth amino acid residues is more preferred, and a position at the eighth or ninth amino acid residue is the most preferred.

The peptides may have a variety of modifications. The modifications may be used to alter or improve the properties of the peptide. For example, peptides disclosed may be those in which one or more amino acids are N-methylated, O-methylated, S-methylated, or C-methylated, or a combination thereof.

The amino and/or carboxy terminals of the peptide may be modified. Amino terminal modification includes methylation (e.g., NHCH₃ or N(CH₃)₂), acetylation (for example, by using acetic acid or a halogenated derivative thereof (e.g., α-chloroacetic acid, α-bromoacetic acid, or α-iodoacetic acid)), addition of a benzyloxycarbonyl (Cbz) group, or blocking an amino terminus with any blocking group including a carboxylate functionality defined by RCOO- or a sulfonyl functionality defined by R-SO₂- (where R is selected from the group consisting of alkyl, aryl, heteroaryl, alkylaryl, etc.), and with a similar group. A person skilled in the art may also make the peptide incorporate a des-amino acid at the N-terminus (so that there is no N-terminal amino group) to reduce sensitivity to proteases or to restrict the conformation of the peptide compound. In a preferred embodiment, the N-terminus is acetylated with acetic acid or acetic anhydride.

Modification of the carboxy terminus includes a step of replacing a free acid with a carboxamide group, or a step of forming a cyclic lactam at the carboxy terminus to introduce structural restraint. A person skilled in the art may also cyclize the peptide disclosed or incorporate a desamino or descarboxy residue at the terminus of the peptide disclosed so that no terminal amino or carboxyl group exists to reduce sensitivity to proteases or restrict the conformation of the peptide. Examples of functional groups at the C-terminus of the peptide disclosed include amides, lower alkyl amides (amide lower alkyl), di (lower alkyl) amides (amide di(lower alkyl)), lower alkoxy, hydroxy, carboxy, and lower ester derivatives thereof, as well as pharmaceutically acceptable salts thereof.

A person skilled in the art can replace a naturally occurring side chain of a genetically encoded amino acid (or a D amino acid as a stereoisomer) with another side chain, for example, with a group (e.g., an alkyl group, a lower (C_{1 to 6}) alkyl group, a cyclic four-membered, five-membered, six-membered, or seven-membered alkyl group, an amide group, a lower alkylamide group, a di(lower alkyl) amide group, a lower alkoxy group, a hydroxy group, or a carboxy group as well as a lower ester derivative thereof), and with a four-membered, five-membered, six-membered, or seven-membered heterocyclic group. In particular, a proline analog in which the ring size of the proline residue is changed from five members to four, six, or seven members may be used. The cyclic group may be saturated or unsaturated, and when the cyclic group is unsaturated, it may be aromatic or non-aromatic. The heterocyclic group preferably comprises one or more nitrogen, oxygen, and/or sulfur heteroatoms. Examples of such a group include furazanyl, furyl, imidazolidinyl, imidazolyl, imidazolinyl, isothiazolyl, isoxazolyl, morpholinyl (e.g., morpholino), oxazolyl, piperazinyl (e.g., 1-piperazinyl), piperidyl (e.g., 1-piperidyl, piperidino), pyranyl, pyrazinyl, pyrazolidinyl, pyrazolinyl, pyrazolyl, pyridazinyl, pyridyl, pyrimidinyl, pyrrolidinyl (e.g., 1-pyrrolidinyl), pyrrolinyl, pyrrolyl, thiadiazolyl, thiazolyl, thienyl, thiomorpholinyl (e.g., thiomorpholino), and
triazolyl. These heterocyclic groups may be substituted or unsubstituted. When a group is substituted, the substituent can be alkyl, alkoxy, halogen, oxygen, or substituted or unsubstituted phenyl.

A person skilled in the art can also readily modify the peptide by phosphorylation and other methods [as described, for example, in Hruby, et al. (1990) Biochem J., 268: pp. 249-262] .

### (Linker)

Binding between the amino acid residue (preferably the side chain of the amino acid residue) at any position of the peptide capable of selectively binding to a tumor vascular endothelial cell and ¹⁰B (preferably a ¹⁰B-containing group) may be via a linker as described above. Examples of the linker include a linker having 1 to 20 carbon atoms (preferably 2 to 15 carbon atoms, more preferably 3 to 10 carbon atoms) which may or may not contain a keto group, an ether bond, a thioether bond, an amide bond, a divalent succinimide group and/or a divalent maleimide group.

Firstly, examples of the bond between the ¹⁰B-containing group and the linker include the following examples (6) to (9), but the present invention is not limited thereto: (6) an amide bond formed of the [10B]boronophenylalanine group represented by the formula (a) or the [18F]fluoro[10B]boronophenylalanine group represented by the formula (c) and an amino group of any linker; (7) an ester bond formed of the [10B]boronophenylalanine group represented by the formula (a) or the [18F]fluoro[10B]boronophenylalanine group represented by the formula (c) and a hydroxy group of any linker; and (8) a thioether bond formed of the [10B]boronocaptate group represented by the formula (b) and a maleimide group of any linker.

(9) An amino group can be introduced into the terminus of the [10B]boronophenylalanine group represented by the formula (a) or the [18F]fluoro[10B]boronophenylalanine group represented by the formula (c) by forming an ester bond using the [10B]boronophenylalanine group represented by the formula (a) or the [18F]fluoro[10B]boronophenylalanine group represented by the formula (c) and any aminoalkyl alcohol (preferably an aminoalkyl alcohol with one to five carbon atoms). Subsequently, any linker having an N-hydroxysuccinimide-activated ester group can be reacted with the [10B]boronophenylalanine group represented by the formula (a) in which an amino group has been introduced at the terminus or the [18F]fluoro[10B]boronophenylalanine group represented by the formula (c) in which an amino group has been introduced at the terminus to bind the linker and the [10B]boronophenylalanine group or the [18F]fluoro[10B]boronophenylalanine group via an amide bond. The amide bond, the thioether bond, the thioester bond, the ester bond, and the disulfide bond can be formed by any organic chemical method.

Subsequently, examples of the bond between an amino acid residue at any position of the peptide capable of selectively binding to a tumor vascular endothelial cell and the linker include the following (11) to (14):
(11) an amide bond between an N-hydroxysuccinimide activated ester group of any linker and a lysine residue which may exist in any position of the peptide and which has an amino group in the side chain;
(12) a thioether bond between a maleimide group of any linker and a cysteine residue which may exist in any position of the peptide and which has a thiol group in the side chain;
(13) a thioester bond between an N-hydroxysuccinimide activated ester group of any linker and a cysteine residue which may exist in any position of the peptide and which has a thiol group in the side chain; and
(14) an ester bond between an N-hydroxysuccinimide activated ester group of any linker and a serine residue or a threonine residue which may exist at any position of the peptide and which has a hydroxy group in the side chain. The amide bond, the thioether bond, the thioester bond, and the ester bond can be formed by any organic chemical method.

Examples of preferred linkers that link ¹⁰B (preferably a ¹⁰B-containing group) to an amino acid residue at any position (preferably the side chain of the amino acid residue) of the peptide capable of selectively binding to a tumor vascular endothelial cell include linkers represented by the following formula (i) or (ii): 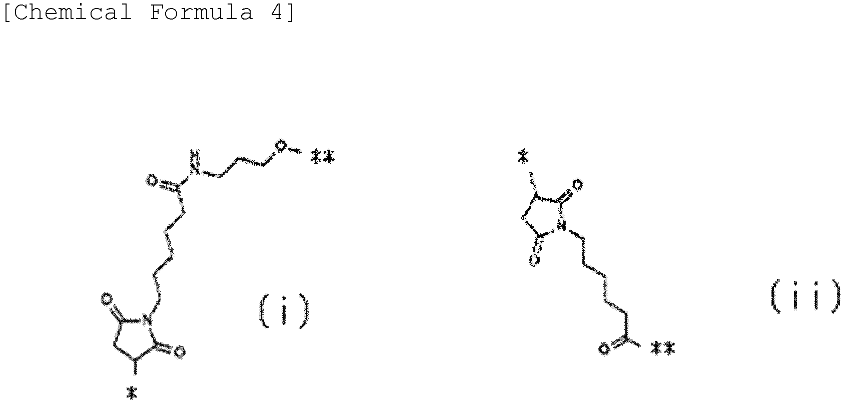 wherein * and ** each represent a bond, and the present invention is, however, not limited thereto.

It is preferable that the bond * in the formulas (i) and (ii) forms a thioether bond with a cysteine residue at any position of the peptide, or a thioether bond with a [10B]borocaptate group represented by the formula (b). It is preferable that the bond ** in the formulas (i) and (ii) forms an amide bond with a lysine residue at any position of the peptide, or an ester bond with a [10B]boronophenylalanine group represented by the formula (a) or an [18F]fluoro[10B]boronophenylalanine group represented by the formula (c). It is also preferable that the bond ** in the formulas (i) and (ii) forms a thioester bond with a cysteine residue at any position of the peptide, or a thioester bond with a [10B]borocaptate group represented by the formula (b).

In actively growing cancer cells, metabolism of phenylalanine is enhanced, and phenylalanine cannot be synthesized in the cells. Therefore, it is known that cancer cells take up and utilize a large amount of phenylalanine in the blood. Therefore, phenylalanine compounds derived from the [10B]boronophenylalanine group represented by the formula (a) or [18F]fluoro[10B]boronophenylalanine group (for example, the formula (c)), etc. (by liberation, etc.) can also be taken up in a large quantity by cancer cells. Therefore, it is preferable that a phenylalanine compound is liberated from the compound by the action of an esterase, etc., which generally exists in cells, so that the phenylalanine compound can move to the cell nucleus where a greater cancer cell-killing effect is expected. From this viewpoint, it is preferable that the [10B]boronophenylalanine group represented by the formula (a) or an [18F]fluoro[10B]boronophenylalanine group (e.g., formula (c)) binds to a linker via an ester bond, or the [10B]boronophenylalanine group represented by the formula (a) or the [18F]fluoro[10B]boronophenylalanine group (e.g., formula (c)) binds to a peptide capable of selectively binding to a tumor vascular endothelial cell via a linker including an ester bond. It is more preferable that the [10B]boronophenylalanine group represented by formula (a) or the [18F]fluoro[10B]boronophenylalanine group (e.g., formula (c)) is bound by the bond ** in the formula (i) to the peptide capable of selectively binding to a tumor vascular endothelial cell via the linker represented by the formula (i).

On the other hand, the [10B]borocaptate compound (e.g., a dissociated product or separated product of [10B]borocaptate group represented by the formula (b)) has 12 ¹⁰B per molecule of the [10B]borocaptate compound and is expected to have a large cancer cell-killing effect by BNCT. Meanwhile, the [10B]borocaptate compound per se is generally difficult to be taken up by cells and is known to remain around cancer cells. Thus, in the case of the [10B]borocaptate group in which the ¹⁰B-containing group is represented by the formula (b), it is preferable that the peptide capable of selectively binding to a tumor vascular endothelial cell and the [10B]borocaptate group represented by the formula (b) are linked via a linker that is not easily cleaved by the action of esterase, etc., which is generally present in cells, (preferably, a linker that does not contain an ester bond). It is more preferable that the [10B]borocaptate group represented by the formula (b) is bound by the bond * in the formula (ii) and thereby the [10B]borocaptate group binds to the peptide capable of selectively binding to a tumor vascular endothelial cell via the linker represented by the formula (ii).

Preferred examples of the compound containing ¹⁰B and a peptide capable of selectively binding to a tumor vascular endothelial cell in the present invention include a compound containing a structure represented by the following formula (i-1) or (ii-1) but are not limited thereto in the present invention. 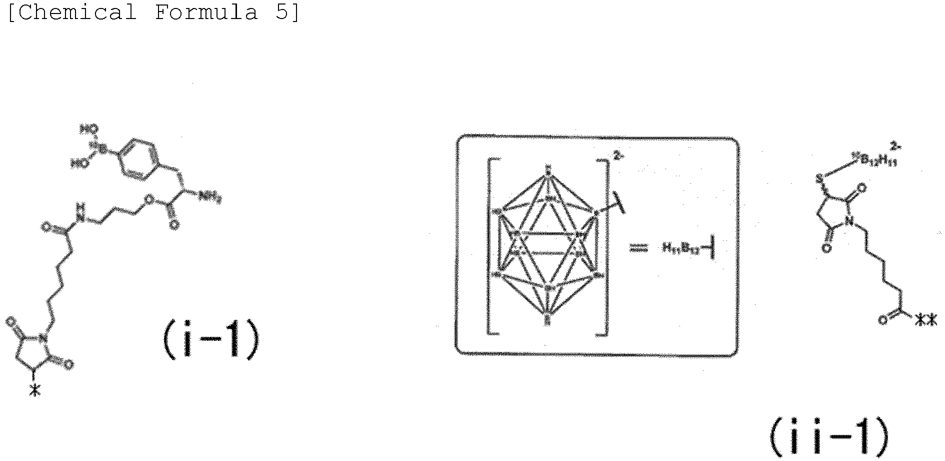 In which * and ** each represent a bonding site with the peptide capable of selectively binding to a tumor vascular endothelial cell. More preferred examples of the compound containing ¹⁰B and a peptide capable of selectively binding to a tumor vascular endothelial cell in the present invention include a compound represented by the following formula (1) or (2), but are not limited thereto in the present invention.

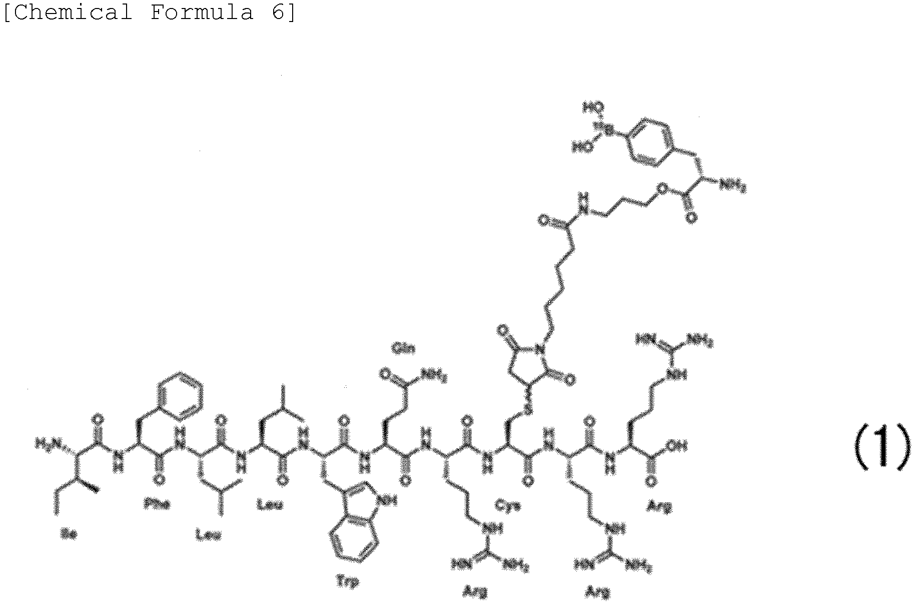

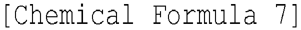

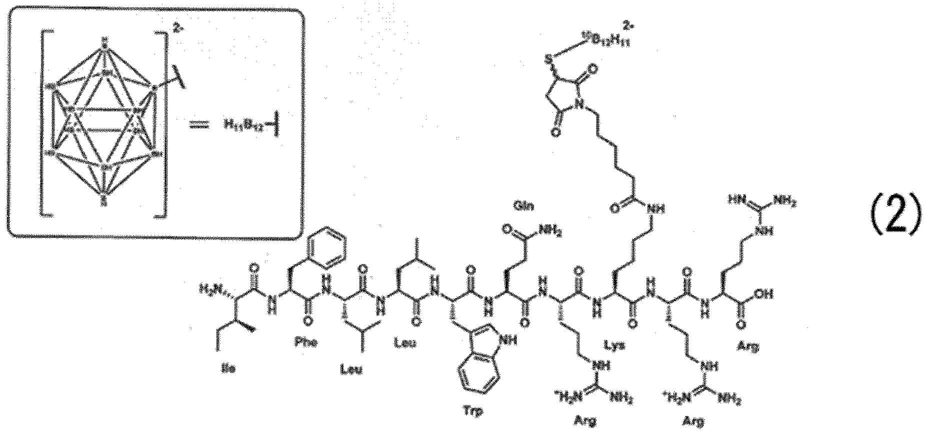

### <Other Components>

The ¹⁰B drug of the present invention may or may not include a pharmaceutically acceptable carrier. The term "pharmaceutically acceptable" means that a material is not a biologically non-desired material nor another non-desired material. That is, the material may be administered to a subject together with the anticancer drug described above without causing any undesired biological action or without interacting in a harmful manner with any of the other components of the anticancer drug described above in which the material is contained. The carrier is, of course, selected as is well known to those skilled in the art to minimize any degradation of an active ingredient (the compound described above) and to minimize any adverse side effects in the subject described above. The materials may be present in a solution or a suspension (e.g., incorporated into microparticles, liposomes, or cells).

Suitable carriers include those described in Remington: The Science and Practice of Pharmacy (19th ed.) ed. A.R. Gennaro, Mack Publishing Company, Easton, PA 1995. Typically, a suitable amount of a pharmaceutically acceptable salt is used in the anticancer drug to render the anticancer drug isotonic. Examples of the pharmaceutically acceptable carriers include, but are not limited to, saline, Ringer's solution, and a dextrose solution. The pH of the solution is preferably from about 5 to about 8, and more preferably from about 7 to about 7.5. Further carriers include sustained release preparations (e.g., a semipermeable matrix of a solid hydrophobic polymer comprising an antibody, wherein the matrix is in the form of a molded article (e.g., a film, a liposome, or microparticles). It will be apparent to those skilled in the art that certain carriers may be more preferred, e.g., depending on the route of administration and the concentration of the composition to be administered.

Pharmaceutical carriers are known to those skilled in the art. These are most typically standard carriers for the administration of drugs to humans, including solutions (e.g., sterile water, saline, and buffered solutions of physiological pH) .

In addition to the compounds generally preferred above, the ¹⁰B drug of the present invention may include carriers, thickeners, diluents, buffers, preservatives, and surfactants, and the like.

### <Use>

The anticancer drug of the present invention is suitable for BNCT because it can selectively accumulate rapidly in tumor tissue at a low dosage amount, in a high concentration. Irradiation with neutrons in BNCT is preferably performed within 60 minutes after the administration, more preferably within 40 minutes after the administration, more preferably within 30 minutes after the administration, and most preferably within 20 minutes after the administration. Although the lower limit value of the start of neutron irradiation is not particularly limited, the starting time is 5 minutes after the administration, and preferably 10 minutes after the administration. Although the irradiation dose (flux) of neutrons is not particularly limited, experiments are carried out in the range of 2 × 10⁶/cm²·s or more (preferably 1 × 10⁷/cm²·s or more, more preferably 1 × 10⁸/cm²·s or more, more preferably 1 × 10⁹/cm²·s or more, more preferably 1 × 10¹⁰/cm²·s or more, more preferably 1 × 10¹¹/cm²·s or more, and most preferably 1 × 10¹²/cm²·s or more). From the viewpoint of PGA measurement sensitivity, flux equal to or higher than this improves the resolution. Therefore, although the upper limit of flux is not particularly limited, for example, 1 × 10¹³/cm²·s or less can be mentioned. 8 × 10¹²/cm²·s or less is preferable, 6 × 10¹²/cm²·s or less is more preferable, and 5 × 10¹²/cm²·s or less is the most preferable.

Although physical dose of neutrons is not particularly limited, for example, a range of 5E⁻¹ Gy or more can be mentioned as a total of thermal neutrons, epithermal neutrons, fast neutrons, and gamma rays. Although the upper limit of the physical dose is not particularly limited, for example, 5 Gy or less can be mentioned as the total.

### <Target>

Although cancers to which the present ¹⁰B drug may be applied are not particularly limited, examples thereof include: lymphomas (Hodgkin and non-Hodgkin), carcinomas, carcinomas of solid tissue, squamous epithelium carcinomas, adenocarcinomas, sarcomas, gliomas, high grade glioma, blastomas, neuroblastomas, plasmacytomas, histiocytomas, melanomas, adenomas, hypoxic tumors, myelomas, AIDS related lymphomas or sarcomas, metastatic cancers, and cancers in general.

More specifically, the following can be mentioned: lymphoma, B-cell lymphoma, T-cell lymphoma, mycosis fungoides, Hodgkin's disease, myelogenic leukemia, bladder cancer, brain cancer, nervous system cancer, head and neck cancer, squamous epithelium carcinomas in the head and neck, kidney cancer, lung cancer (e.g., small cell lung cancer and non-small cell lung cancer), neuroblastoma/glioblastoma, ovarian cancer, pancreatic cancer, prostate cancer, skin cancer, liver cancer, melanoma, squamous epithelium carcinomas in the mouth, throat, larynx, and lung, colon cancer, cervical cancer, cervical carcinoma, breast cancer, epithelial cancer, kidney cancer, urinary and genital organ cancer, pulmonary cancer, esophagus cancer, gastric cancer, head and neck cancer, colon cancer, hematopoietic cancer, testicular cancer, colon and rectal cancer, or pancreatic cancer, etc.

### EXAMPLES

Below, the present invention is described in more detail with reference to the Examples, but the scope of the present invention is not limited to these Examples.

### <Synthetic Example 1>

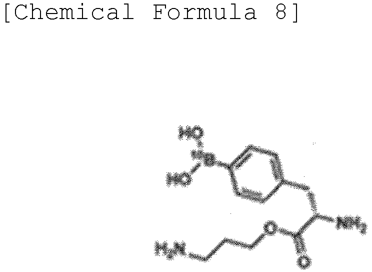

The peptide represented by the amino acid sequence IFLLWQRCRR (SEQ ID NO: 2) was chemically synthesized using a 9-fluorenylmethoxycarbonyl method (Fmoc method). 2,000 mg of the peptide obtained and 3,000 mg of the compound represented by the above formula were mixed, and N-(6-maleimidecaproyloxy)succinimide (EMCS) was added dropwise to crosslink between a thiol group of cysteine of the peptide and a terminal amino group of the compound represented by the formula with the EMCS to obtain 420 mg of a compound represented by the following formula (1) (hereinafter, simply referred to as "IF7-¹⁰BPA").

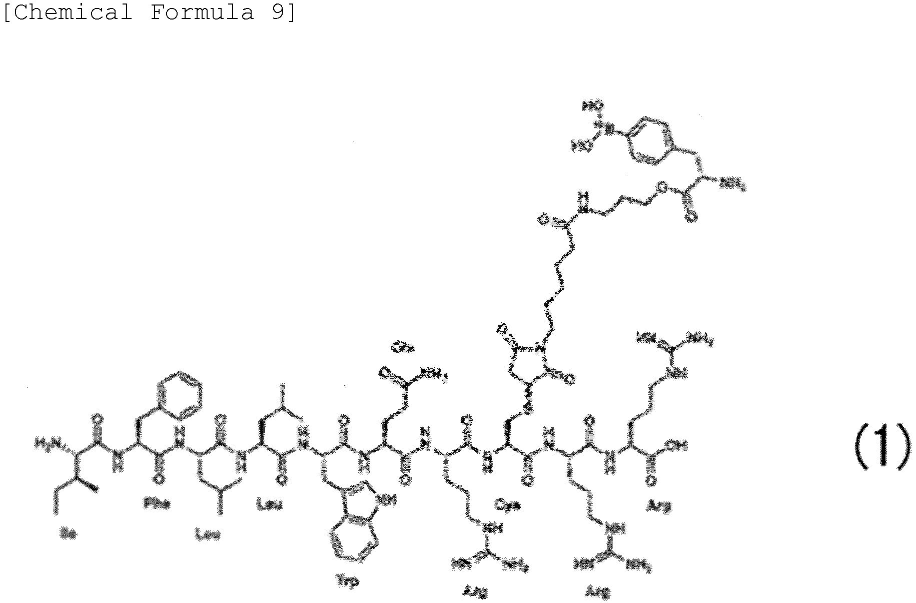

### <Synthesis Example 2>

The peptide represented by the amino acid sequence IFLLWQRKRR (SEQ ID NO: 3) was chemically synthesized using tert-butoxycarbonyl method (Boc method). 2,000 mg of the peptide obtained and 3,000 mg of sodium [10B]borocaptate were mixed, and N-(6-maleimidocaproyloxy)succinimide (EMCS) was added dropwise to crosslink between an amino group of lysine of the peptide and a thiol group of the sodium [10B]borocaptate with the EMCS to obtain 415 mg of a compound represented by the following formula (2) (hereinafter, simply referred to as "IF7-¹⁰BSH").

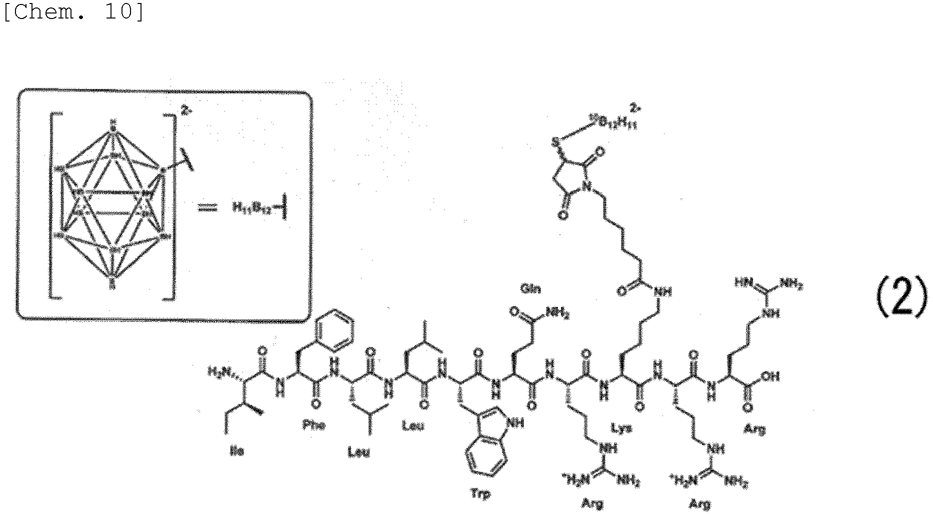

### [Tumor Accumulation Ability Test]

### <Examples 1 and 2>

Tumor accumulation ability was tested in tumor-bearing mice (7 mm in tumor diameter) seeded on the thigh with a murine bladder cancer cell MBT2, with respect to IF7-¹⁰BPA synthesized in Synthesis Example 1 above and IF7-¹⁰BSH synthesized in Synthesis Example 2 above. 100 µL (175 µg) of IF7-¹⁰BPA or IF7-¹⁰BSH adjusted to 7 mg/kg was administered to the tail vein of the tumor-bearing mice. ¹⁰B levels (ppm) in the organs of the tumor-bearing mice were measured 5, 10, and 20 minutes after the tail-vein administration by the PGA-method.

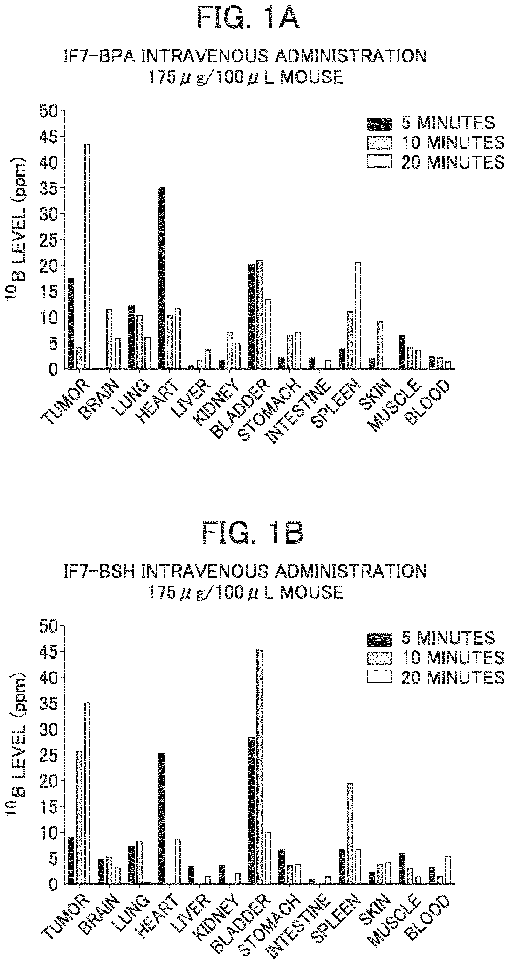

FIGs. 1A and 1B are drawings indicating ¹⁰B levels (ppm) in the organs of the tumor-bearing mice 5, 10, and 20 minutes after tail-vein administration of 100 µL (175 µg) of the IF7-¹⁰BPA and IF7-¹⁰BSH, respectively. As is clear from the results shown in FIGs. 1A and 1B, it can be seen that for both IF7-¹⁰BPA and IF7-¹⁰BSH, ¹⁰B levels were less than 20 ppm in the brain, lung, heart, liver, kidney, bladder, stomach, intestine, spleen, skin, muscles, and blood 20 minutes after the administration, whereas ¹⁰B specifically accumulated in the tumor tissue of the tumor-bearing mice at a level of at least 25 ppm, 20 minutes after the administration. Thus, both IF7-¹⁰BPA and IF7-¹⁰BSH could selectively accumulate in tumor tissue at a low dosage amount (7 mg/kg) rapidly (20 min) at a high level (≥ 25 ppm), indicating that IF7-¹⁰BPA and IF7-¹⁰BSH are suitable as a ¹⁰B drug for BNCT.

### <Comparative Example 1>

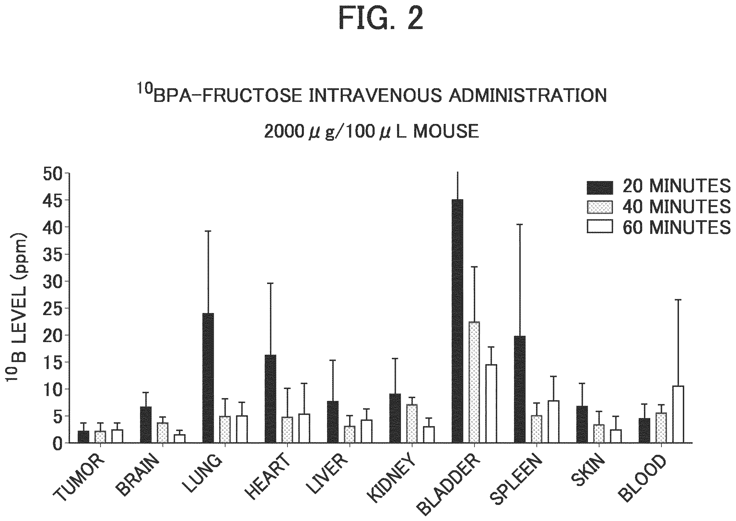

Additionally, as Comparative Example 1, a tumor accumulation ability test was similarly performed using β-D-fructopyranose, 2' position of which is esterified with [10B]boronophenylalanine group represented by the formula (a) (hereinafter, simply referred to as "¹⁰BPA-fructose"). 100 µL (2,000 µg) of ¹⁰BPA-fructose whose dosage amount was increased to 100 mg/kg was administered to the tail vein of tumor-bearing mice. ¹⁰B levels (ppm) in the respective organs of the tumor-bearing mice were measured 20, 40, and 60 minutes after the tail-vein administration by ICP-AES method. FIG. 2 is a drawing indicating ¹⁰B levels (ppm) in the organs of the tumor-bearing mice at 20, 40, and 60 minutes after tail-vein administration of 100 µL (2,000 µg) of ¹⁰BPA-fructose. As is clear from the results shown in FIG. 2, ¹⁰BPA-fructose did not accumulate at all in the tumor tissue even after 20, 40, and 60 minutes elapsed after the administration. It can be seen that ¹⁰BPA-fructose did not significantly discriminate the tumor tissue from the other organs.

### <Example 3 and Comparative Examples 2 and 3>

### (Example 3)

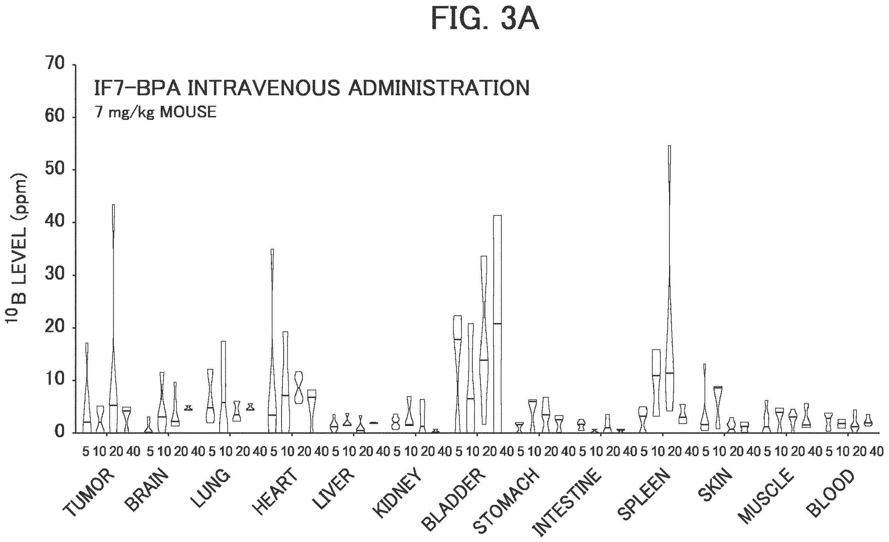

¹⁰B levels in the respective organs of the tumor-bearing mice were measured in the same manner as in Example 1, except that the measurement was performed 40 minutes after the administration in addition to 5, 10 and 20 minutes, the number of n in the respective data was 3 or more, and the measurement results were indicated in violin plots. The results are indicated in FIG. 3A.

### (Comparative Example 2)

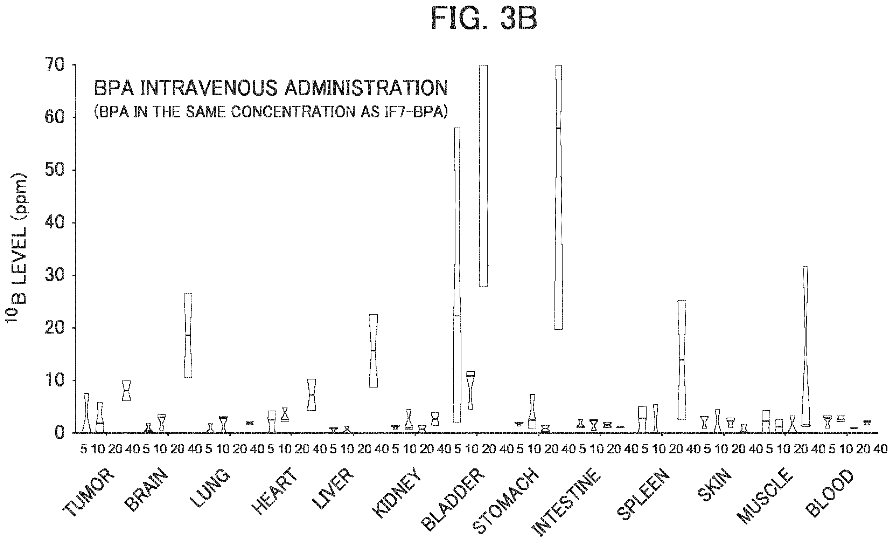

¹⁰B levels in the respective organs of the tumor-bearing mice were measured in the same manner as in Example 3, except that instead of IF7-¹⁰BPA, [10B]boronophenylalanine (¹⁰BPA) in the same concentration was administered. The results are indicated in violin plots in FIG. 3B.

### (Comparative Example 3)

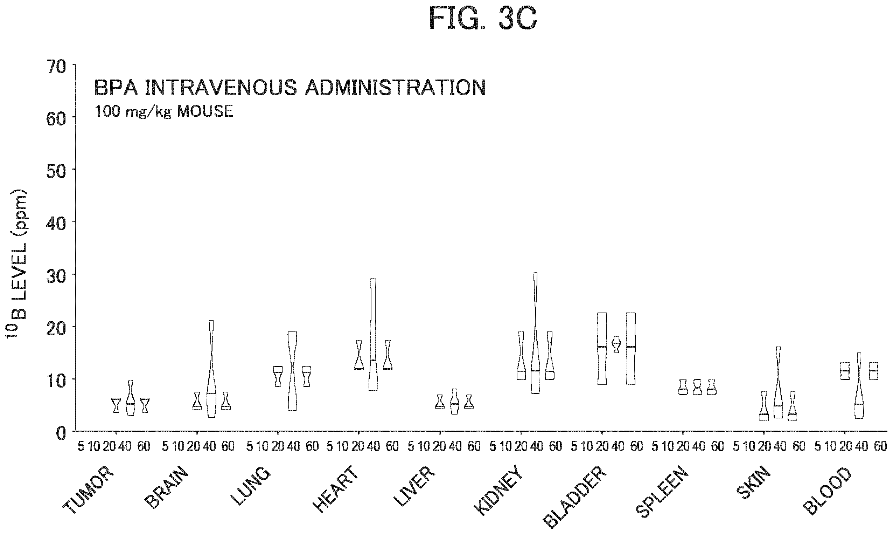

¹⁰B levels in the respective organs of the tumor-bearing mice were measured in the same manner as in Comparative Example 2, except that the concentration of ¹⁰BPA was changed from 7 mg/kg to 100 mg/kg. The results are indicated in violin plots in FIG. 3C.

### (Results)

As is clear from the results shown in FIGs. 3A to 3C, it can be seen that in Example 3 in which IF7-¹⁰BPA was used, accumulation of ¹⁰B in the tumor tends to occur in a shorter time after the administration than in the Comparative Examples 2 and 3 in which ¹⁰BPA was used, in particular 20 minutes after the administration. Further, even if compared to Comparative Example 3, in which the concentration of ¹⁰BPA was 100 mg/kg, ¹⁰B level in the tumor in Example 3 in which IF7-¹⁰BPA was used can be said to be not inferior.

### <Example 4 and Comparative Example 4>

### (Example 4)

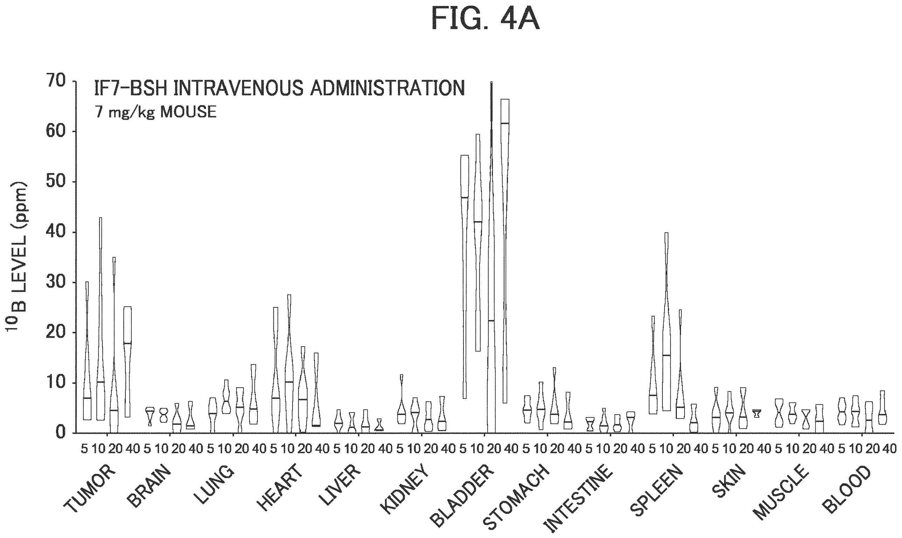

¹⁰B levels in the respective organs of the tumor-bearing mice were measured in the same manner as in Example 2, except that the measurement was performed 40 minutes after the administration in addition to 5, 10, and 20 minutes, the number of n in the respective data was 3 or more, and the measurement results were indicated in violin plots. The results are indicated in FIG. 4A.

### (Comparative Example 4)

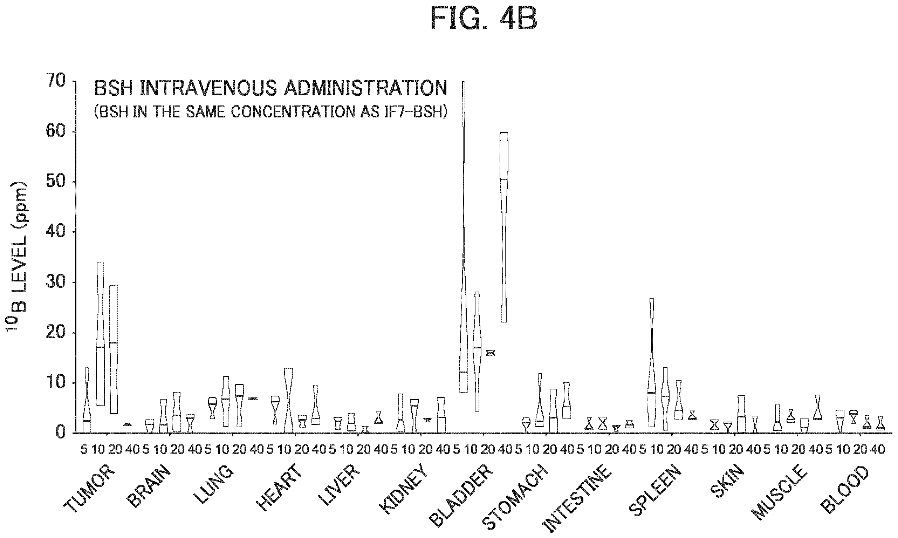

¹⁰B levels in the respective organs of the tumor-bearing mice were measured in the same manner as in Example 4, except that instead of IF7-¹⁰BSH, mercaptoundecahydrododeca [10B]borate (¹⁰BSH) in the same concentration was administered. The results are indicated in FIG. 4B in violin plots.

### (Results)

As is clear from the results shown in FIGs. 4A and 4B, it can be seen that in Example 4 in which IF7-¹⁰BSH was used, accumulation of ¹⁰B in the tumor tends to occur 20 minutes to 40 minutes after the administration, in the same or higher levels compared to Comparative Example 4 in which ¹⁰BSH was used.

### [Neutron Irradiation Test]

### <Examples 5 and 6>

### (Example 5)

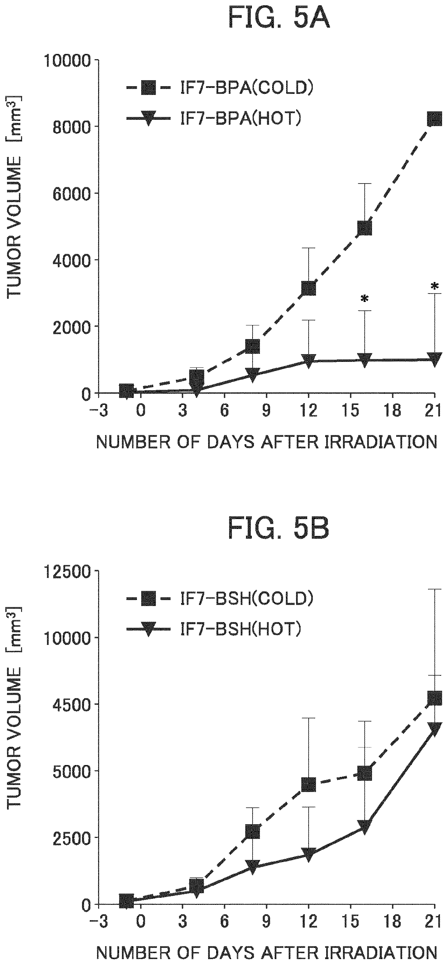

IF7-¹⁰BPA was administered to the tail vein of tumor-bearing mice seeded in the thigh with murine bladder cancer cell strain MBT2 at a dosage amount of 7 mg/kg. Thereafter, tumor volumes (mm³) thereof were measured for 21 days, with respect to a group (hot group), which was irradiated 40 minutes after the tail-vein administration with neutrons for 30 minutes under the following conditions and a group (cold group), which was not irradiated. The results are indicated in FIG. 5A. In the drawing, * represents p < 0.05.
(1) Neutron fluence (cm⁻²·s⁻¹)
   2.1E⁺¹² (2.1 × 10¹²) to 4.6E⁺¹²
(2) Physical dose (Gy)
   Thermal neutrons: 2.8E⁻¹ to 6.1E⁻¹
   Epithermal neutrons: 3.0E⁻² to 6.5E⁻²
   Fast neutrons: 2.1E⁻¹ to 4.6E⁻¹
   Gamma rays: 2.1E⁻¹ to 4.6E⁻¹
   Total: 8.9E⁻¹ to 1.5E⁰

### (Example 6)

Administration was performed in the same manner as in Example 5, except that IF7-¹⁰BSH was administered instead of IF7-¹⁰BPA. Thereafter, tumor volumes (mm³) were measured for 21 days with respect to the hot group which was irradiated with neutrons 40 minutes after the administration and the cold group which was not irradiated. Results are indicated in FIG. 5B.

### (Results)

As is clear from the results indicated in FIGs. 5A and 5B, it can be seen that the effect of reducing the tumor volume was pronounced in both the hot groups after the administration of IF7-¹⁰BPA or IF7-¹⁰BSH than in the cold groups after the administration of IF7-¹⁰BPA or IF7-¹⁰BSH.

### <Example 7 and Comparative Example 5>

### (Example 7)

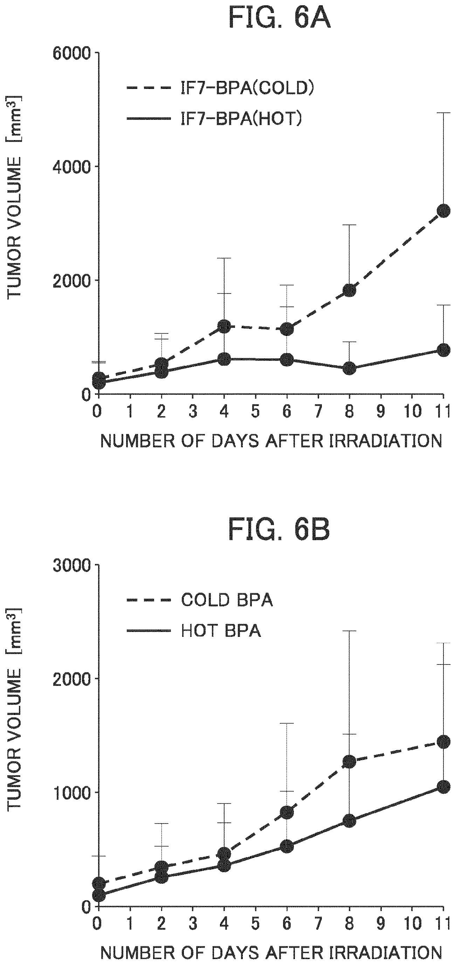

IF7-¹⁰BPA was administered to tail vein of the tumor-bearing mice seeded with murine bladder cancer cell strain MBT2 at a dosage amount of 7 mg/kg. Thereafter, tumor volumes (mm³) were measured for 21 days with respect to a hot group which was irradiated with neutrons 40 minutes after the tail-vein administration and a cold group which was not irradiated. Results are indicated in FIG. 6A. As is clear from the results shown in FIG. 6A, it can be seen that the effect of reducing the tumor volume was more pronounced in the hot group after the administration of IF7-¹⁰BPA than in the cold group after the administration of IF7-¹⁰BPA. Further, it can be said that the effect of reducing the tumor volume was greater in the hot group after the administration of IF7-¹⁰BPA in Example 7 than in the hot group after the administration of [10B]boronophenylalanine (¹⁰BPA) in Comparative Example 5.

### (Comparative Example 5)

The administration was performed in the same manner as in Example 7, except that ¹⁰BPA was administered instead of IF7-¹⁰BPA, tumor volumes (mm³) were measured for 21 days with respect to the hot group irradiated 40 minutes after the administration with neutrons and the cold group which was not irradiated. Results are indicated in FIG. 6B. As is clear from the results indicated in FIG. 6B, though the effect of reducing tumor volumes can be seen in the hot group after the administration of ¹⁰BPA, compared to the cold group after the administration of ¹⁰BPA, the effect is not as pronounced as in Example 7.

### [Sequence Listing]

### PCT19-001 ST25.txt

## Claims

1. An accumulative ¹⁰B drug comprising a compound comprising ¹⁰B and a peptide capable of selectively binding to a tumor vascular endothelial cell, wherein the accumulative ¹⁰B drug is administered to a subject affected with cancer in an amount from 300 to 600 mg/administration and wherein the ¹⁰B drug accumulates in a cancer-affected tissue of the subject after the administration so that a ¹⁰B level therein is 1 ppm or more.

2. The accumulative ¹⁰B drug according to claim 1, wherein the accumulative ¹⁰B drug accumulates so that the ¹⁰B level in the cancer-affected tissue of the subject is 20 ppm or more.

3. The accumulative ¹⁰B drug according to claim 1 or 2, wherein the ¹⁰B level in the cancer-affected tissue is 1 ppm or more during a period from 10 minutes to 30 minutes after the administration of the accumulative ¹⁰B drug.

4. The accumulative ¹⁰B drug according to any one of claims 1 to 3, wherein the administration is injection administration.

5. The accumulative ¹⁰B drug according to any one of claims 1 to 4, wherein the compound contains a ¹⁰B-containing group and the ¹⁰B-containing group is an L-p-[10B]boronophenylalanine group, an [18F]fluoro[10B]boronophenylalanine group, or a [10B]borocaptate group.

6. The accumulative ¹⁰B drug according to any one of claims 1 to 5, wherein the peptide is a peptide capable of selectively binding to annexin 1.

7. An accumulative ¹⁰B drug, comprising a ¹⁰B-containing group and a peptide that can selectively bind to annexin 1, wherein the ¹⁰B-containing group is an L-p-[10B]boronophenylalanine group, an [18F]fluoro[10B]boronophenylalanine group, or a [10B]borocaptate group.

8. The accumulative ¹⁰B drug according to claim 6 or 7, wherein the peptide comprises an amino acid sequence of IFLLWQR (amino acids 1 to 7 of SEQ ID NO: 1), an amino acid sequence of IFLLWQRX (amino acids 1 to 8 of SEQ ID NO: 1), an amino acid sequence of IFLLWQRXX (amino acids 1 to 9 of SEQ ID NO: 1), an amino acid sequence of IFLLWQRXXX (amino acids 1 to 10 of SEQ ID NO: 1), an amino acid sequence of IFLLWQRXXXX (amino acids 1 to 11 of SEQ ID NO: 1), or an amino acid sequence of IFLLWQRXXXXX (amino acids 1 to 12 of SEQ ID NO: 1), wherein each X independently represents a polar or charged amino acid, and wherein one or two amino acids of IFLLWQR in each of the amino acid sequences may be substituted.

9. The accumulative ¹⁰B drug according to any one of claims 1 to 8, wherein the peptide and a ¹⁰B-containing group are linked to each other via a linker.

10. The accumulative ¹⁰B drug according to claim 9, wherein the ¹⁰B-containing group is an L-p-[10B]boronophenylalanine group or an [18F]fluoro[10B]boronophenylalanine group, and wherein the ¹⁰B-containing group is linked to the linker via an ester bond or the linker contains an ester bond.

11. The accumulative ¹⁰B drug according to claim 9, wherein the ¹⁰B-containing group is a [10B]borocaptate group and wherein the linker contains no ester bond.

12. The accumulative ¹⁰B drug according to any one of claims 9 to 11, wherein the linker is a linker represented by the following formula (i) or (ii): 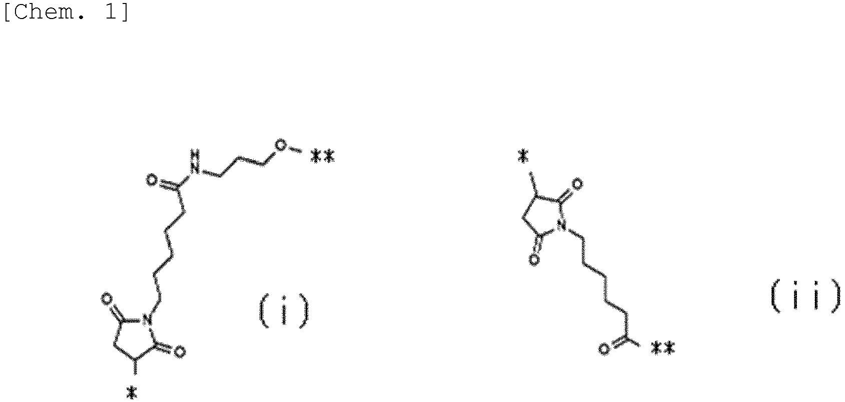 wherein * and ** each represent a bond.

13. The accumulative ¹⁰B drug according to any one of claims 1 to 12, wherein the compound is a compound containing a structure represented by the following formula (i-1) or (ii-1) : 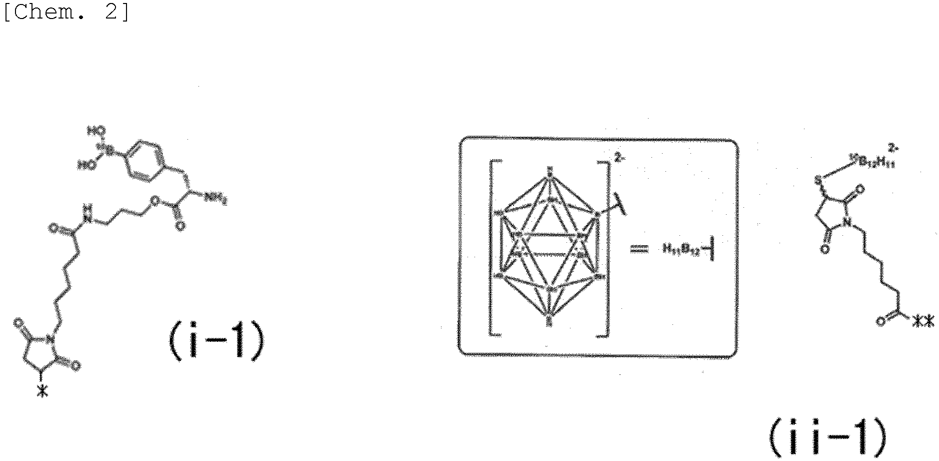 wherein * and ** each represent a bond at which the peptide capable of selectively binding to a tumor vascular endothelial cell binds.

14. The accumulative ¹⁰B drug according to any one of claims 1 to 13, wherein the ¹⁰B drug is for use in BNCT.

15. The accumulative ¹⁰B drug according to claim 14, wherein the ¹⁰B drug is used to perform neutron irradiation within 60 minutes after the administration.

16. The accumulative ¹⁰B drug according to claim 14 or 15, wherein the ¹⁰B drug is used to perform neutron irradiation at a dose of 2 × 10⁶/cm² s or more.

17. The accumulative ¹⁰B drug according to claim 1, wherein a dosage amount per administration to the subject is 5 to 9 mg per unit body weight (1kg) of the subject.
